Europäisches Patentamt

European Patent Office

Office européen des brevets

(19)

(11) Numéro de publication: **0 213 999**
**B1**

# FASCICULE DE BREVET EUROPEEN

(45) Date de publication du fascicule du brevet:
03.01.90

(51) Int. Cl.⁴: **A61K 7/48**

(21) Numéro de dépôt: **86401620.9**

(22) Date de dépôt: **21.07.86**

(54) **Nouvelle composition cosmétique issue de cultures cellulaires de tissu conjonctif.**

(30) Priorité: **01.08.85 FR 8511766**

(43) Date de publication de la demande:
**11.03.87 Bulletin 87/11**

(45) Mention de la délivrance du brevet:
**03.01.90 Bulletin 90/1**

(84) Etats contractants désignés:
**DE GB IT**

(56) Documents cités:
**EP-A- 0 107 885**
**WO-A-81/00260**
**US-A- 3 660 566**
**US-A- 3 699 963**

**Le dossier contient des informations techniques présentées postérieurement au dépôt de la demande et ne figurant pas dans le présent fascicule.**

(73) Titulaire: **Seguin, Marie-Christine, Périgord 1 6 Lacets Saint-Léon, Monte-Carlo(MC)**
Titulaire: **Gueyne, Jean, Périgord 1 6 Lacets Saint Léon, Monte-Carlo(MC)**

(72) Inventeur: **Seguin, Marie-Christine, Périgord 1 6 Lacets Saint-Léon, Monte-Carlo(MC)**
Inventeur: **Gueyne, Jean, Périgord 1 6 Lacets Saint Léon, Monte-Carlo(MC)**

(74) Mandataire: **Kohn, Armand, 5 Avenue Foch, F-92380 Garches(FR)**

ACTORUM AG

## Description

L'invention concerne l'utilisation, comme composition cosmétique, pour le traitement de la peau, d'un milieu nutritif de cellules de la peau; et comprend aussi une nouvelle composition et sa préparation; cette utilisation et composition étant destinées à l'amélioration de l'aspect et de la qualité de la peau. L'invention comprend en outre un article cosmétique, pour le traitement de la peau, qui contient un milieu nutritif de cellules de la peau.

L'amélioration de la qualité de la peau constitue un des objectifs importants de la cosmétologie. Il est, en effet, souvent nécessaire de stimuler la régénération naturelle de la peau, en dehors de sa coloration, de son hydratation, de sa nutrition ou de sa protection contre les rayons solaires ou les vents. Aussi existe-t-il un grand nombre de crèmes, le lotions, et de laits à base de différents produits naturels ou synthétiques, employés en vue de la stimulation des processus normaux, pour rendre à une peau, plus ou moins abîmée ou vieillissante, ses souplesse, tenue et aspect agréable. Ainsi, depuis les différentes matières grasses, végétales et animales, telles que, par exemple, les huiles d'amande douce ou de palme, et les graisses de crinière de cheval ou de vison, dont certaines sont utilisées depuis des siècles, jusqu'aux compositions à base d'extraits d'élastine, de collagène, de placenta, de végétaux et vitamines, un grand nombre de produits ont été expérimentés avec des résultats fort variables. Lorsqu'il s'agit de redonner à la peau, véritablement, les vigueurs, souplesse et éclat, les compositions employées jusqu'à présent sont en défaut.

La présente invention crée, dans ce domaine, un perfectionnement important : elle intervient sur les mauvais état et aspect de la peau, par le moyen d'une composition cytologi que, dont le rôle est de transmettre à la peau des vecteurs cellulaires, dans un environnement de croissance ; ces vecteurs exercent une action stimulatrice de la régénération tissulaire voire une action réparatrice. Ainsi, suivant l'invention, n'est-il plus question, comme c'est le cas généralement dans l'art connu, d'apporter à la peau des matières étrangères, mais d'y induire des phénomènes naturels de croissance au moyen de facteurs spécifiques de la peau elle-même. Bien que la publication WO-A-81/00260 ait décrit l'utilisation de fibroblastes pour le traitement de l'épiderme, il s'agissait là de fibroblastes très spéciaux, ceux de souris 3T3 irradiées, pui ont un effet inhibiteur sur la croissance des fibroblastes humains; d'ailleurs, selon cette publication, les cellules de la peau adulte ne sont pas dédoublables, alors que suivant la présente invention on réussit bien à les régénérer.

L'utilisation, suivant l'invention, comme composition cosmétique pour le traitement de la peau, d'un milieu nutritif de cellules de la peau, est caractérisé en ce qu'une culture de tissu conjonctif, particulièrement de fibroblastes, se trouve dans ce milieu nutritif. Bien que les cellules de la culture puissent être humaines, animales despécifiées, ou même végétales, vivantes ou mortes, des résultats particulièrement favorables sont obtenus avec des cultures de fibroblastes et kératinocytes humains. Il peut être intéressant d'employer une culture de fibroblastes obtenue à partir d'un explant des cellules correspondantes, prélevé sur le sujet auquel est justement destinée la composition cosmétique donnée, suivant l'invention.

Le procédé d'obtention de la composition suivant l'invention peut être réalisé par une culture cellulaire selon la technique suivante. Des fibroblastes humains sont isolés à partir de fragments de la peau, et débarrassés du tissu graisseux subcutané. Les fragments de 1 mm³ sont placés dans des boîtes de culture et recouverts de 1 ml de milieu DMEM (Eagle) complété avec du sérum de veau foetal à 10%, 2 mM du glutamine, 1 mM de pyruvate de sodium, acides aminés non-essentiels et 100 unités/ml de pénicilline-streptomycine. Les cultures sont incubées à 37°C, en atmosphère humide de 95% d'air et 5% de $CO_2$.

Les fibroblastes croissent à partir de l'explant; ils peuvent être repiqués après 2-3 semaines. Les cellules sont détachées quantitativement de la boîte de culture après 5 mn d'exposition à une solution de trypsine, et recueillies après centrifugation à 1700 tr/mn. Elles sont mises en suspension dans le milieu de culture et distribuées dans les supports de culture à une densité initiale de $1 \times 10^2$ à $1 \times 10^8$ cellules.

Le milieu nutritif employé peut être liquide, pâteux, particulaire ou solide; il peut par exemple être sous la forme d'un sérum physiologique, d'eau distillée, d'un gel avec de la gélatéine ou d'une autre matière gélifiante, de micro billes de verre traité, de poudre inerte, par exemple un polymère : PVC micronisé à 20 microns, fibres de polyester, film de collagène etc.

Le support cellulaire, entrant dans la nouvelle composition cosmétique, peut contenir un nombre très variable de cellules, par exemple $10^2$ à $10^8$ par ml, et de préférence environ $10^4$ à $10^8$.

Bien que la composition puisse être constituée par des cellules de tissu conjonctif ou autres cellules, telles que prélevées sur un sujet donné, mises en suspension dans le milieu convenable pour leur culture, la forme préférée de l'invention réside en une culture, dans laquelle la croissance des cellules initiales a au moins été multipliée par 10. Des compositions particulièrement efficaces renferment environ 10 à 100 fois le nombre de cellules d'ensemencement de départ.

Les fibroblastes et les kératinocytes, étant les cellules de choix pour la composition suivant l'invention, peuvent être prélevées sur la peau de tout endroit du corps humain, animal ou végétal. Chez l'homme, il est pratique de faire le prélèvement d'une très petite quantité de peau au lobe d'une oreille ou une cuisse par exemple. Les explants dans les supports de cultures sont maintenus ainsi à 37°C, jusqu'à ce que leur nombre se multiplie dans les proportions voulues, notamment comme indiqué plus haut.

Après cette phase de croissance et prolifération, quelle que soit la concentration en cellules dans le milieu de culture, si les cellules sont mortes, la composition doit être conservée au froid, de préférence entre 1° et 6°C, le mieux à 4°C ; si elles sont vivantes, la composition est à conserver à 37°C ou - 80°C. Si le support de culture est liquide, la composition peut être conditionnée dans des tubes fermés, flacons, ampoules, comme il est connu de le faire pour divers produits pharmaceutiques et cosmétiques; cependant, selon deux importantes formes d'exécution, la nouvelle composition cosmétique est conservée dans un dispositif spécial qui, tout en assurant une excellente conservation, facilite l'application de la composition sur la peau de l'usager. Ces dispositifs constituent un article qui fait également partie de l'invention.

Suivant un premier trait de l'invention, l'article est constitué par au moins trois couches superposées, le support nutritif et ces cellules sont dans ce cas sous forme de plaque de très faible épaisseur ou filmogène stériles, par exemple: film d'origine végétale, fibres de lin, de coton, gel d'acide alginique nappeiforme ou animale biologiquement non structuré, film de gélatine ou structuré: collagène; conviennent à cet effet des matières collagénique, cellulosique, polysaccharides, polyéthylènes-glycols, polyvinyl-pyrrolidones, alginates et substances similaires. Ce support peut d'ailleurs être solide, liquide ou pâteux. Très favorables sont les produits qui permettent d'avoir une culture portée par une couche spongieuse, comme par exemple mousse polyvinyl-pyrrolidone ou de copolymères de celle-ci avec de l'acétate de vinyle, par de la cellulose spongieuse ou bien par une éponge de gélatine. Particulièrement pratique est la mise de la composition, suivant l'invention, sous la forme d'une feuille souple, par exemple, avec comme support de la gélatine, du collagène, du polyéthylène glycol, du dextrane ou autre composé filmogène du même type. Cette couche cellulaire est insérée entre deux autres couches inertes de protection, dont une peut être enlevée au moment de l'application de celle-ci sur la peau de l'usager. Dans d'autres cas, lors de l'application, on enlève la 1ère couche de protection, on appose la couche cellulaire et la 2ème couche de protection, puis on enlève cette 2ème couche, la couche cellulaire s'intégrant à la peau, en particulier lorsque le support est un film de collagène. On a ainsi un article en forme de feuille, dont les dimensions et forme sont adaptées à la région du corps où doit avoir lieu l'application.

Dans une autre forme d'exécution le support susmentionné se trouve dans un récipient très peu profond, servant de protecteur de l'ensemble et permettant d'avoir éventuellement un support liquide ou pâteux.

Les différentes variantes de l'invention sont décrites plus en détail ci-après, avec référence aux dessins annexés.

Fig. 1 représente la coupe transversale d'une feuille de la composition à trois couches superposées,

Fig. 2 est également une coupe transversale d'un article suivant l'invention, où le support des cellules est logé dans un récipient de très faible profondeur.

Fig. 3 est une vue en plan de l'article selon Fig.2.

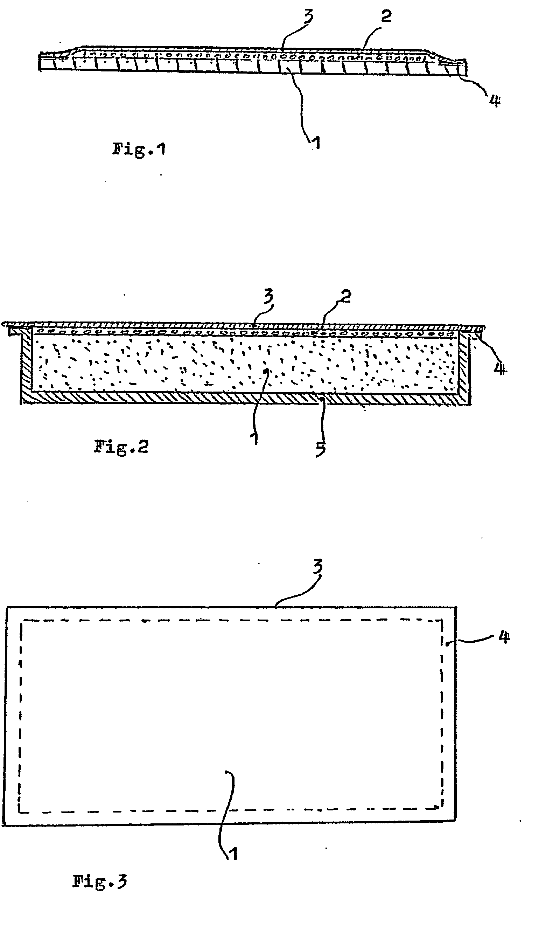

Sur la Fig. 1, on voit la section d'un ensemble formé par un support plat 1 porteur de cellules 2, notamment de fibroblastes, avec leur milieu nutritif de culture incorporé; cette couche active est recouverte elle-même d'un film protecteur 3, susceptible d'être décollé de 1 au moment de l'emploi de la feuille triple 1-2-3. Les films 3 et 1 sont imperméable au milieu de culture des cellules utilisées.

Il est important de choisir pour 1 et 3 des matières appropriées convenant à la culture des cellules employées, sans aucun risque d'altérations de ces dernières.

Comme le support 1 vient au contact de la peau, lors de l'application de l'article, il est souhaitable qu'au moins sa couche superficielle porte ou contienne un nombre suffisamment élevé de fibroblastes, de préférence $10^4$ à $10^8$ cellules par ml.

La couche active, représentée par 2 sur les dessins, est constituée par les cellules, de préférence fibroblastiques, mais pouvant éventuellement être ou comprendre kératinocytes, lymphocytes, etc., dispersés dans le milieu de culture utilisé. On a représenté 2 comme une couche distincte, mais, lorsque le support 1 est suffisamment absorbant vis-à-vis de la culture, celle-ci se trouve, en partie ou en entier, dans la couche formée par le support 1.

Dans une forme d'éxécution particulière, la culture 2 est entièrement mélangée avec la matière du support. C'est le cas du collagène ou d'un produit gélifiant, par exemple polyvinyl-pyrrolidone, gélatine ou polysaccharide, dispersé dans le milieu liquide, contenant les cellules multipliées. Dans ce cas les deux films protecteurs sont identiques ou semblables.

De préférence, le pourtour de la couche 1 est muni d'un adhésif pour permettre le collage de la feuille 1-2 sur la peau du patient, après l'enlèvement du film protecteur 3. Comme il est connu d'utiliser des bandes collantes convenant à la peau humaine, notamment dans des pansements par exemple sous la forme de sparadrap, il n'y a pas lieu de décrire ici la nature de ces rebords collants 4 (fig. 1) appliqués aux articles suivant l'invention.

En ce qui concerne la couche 3, recouvrant la culture, tant que celle-ci n'est pas au contact de la peau à traiter, elle est constituée par une feuille mince de matière souple, de préférence polymère insoluble dans l'eau et inerte vis-à-vis du milieu de culture. Conviennent bien des films de polyéthylène, polypropylène, polyamide, polyacrylates, acétate de cellulose, cellulose et similaires.

Bien que les épaisseurs et les autres dimensions des articles en feuilles, suivant l'invention, puissent varier largement, on utilise avantageusement des produits dont le support 1 a une épaisseur d'environ 0,05 à 1 mm, la couche 2 active 0,1 à 3 mm et le film 3 protecteur 0,05 à 0,5 mm. Les autres dimensions préférées sont de l'ordre de 2 x 5 à 7 x 15 cm pour les largeur x longueur, et plus particulièrement 4 x 7 cm permettant une application aisée sur le visage, les mains, les jambes. Ces feuilles sont polygonales, à bords arrondis et, en particulier d'une forme trapézoïdale permettant de recouvrir la majeure partie de la joue.

Le dispositif selon Fig. 2 et 3 comporte les mêmes couches 1 à 3 que celui de la Fig. 1, mais en général le support 1 y est plus épais et peut être particulaire ou/et liquide. Ce support est logé dans un récipient 5 à faible profonfeur, par exemple de 3 à 15 mm, et de préférence de 5 à 10 mm. On retrouve ici le pourtour 4 encollé, permettant la fixation temporaire, sur la peau, du récipient 5 avec son support 1 porteur des cellules 2, lorsque le film protecteur 3 a été enlevé. Lors de l'utilisation du produit, la composition 1-2 peut être prise du récipient 5, au moyen, par exemple, d'une compresse ; alors la compresse absorbe, si nécessaire, l'excès de la phase liquide présente, et - chargée des cellules 2 - est apposée sur la peau, procurant ainsi le contact des cellules 2 avec cette dernière.

Un autre article suivant l'invention, comprenant la nouvelle composition, est constitué par un récipient, part exemple tube rigide ou souple, muni d'un applicateur, chargé de composition. Cela peut être notamment une serinque de capacité appropriée. De préférence un tel article comporte un tube-dose, évitant des utilisations multiples, susceptibles d'entraîner la pollution toujours possible, surtout avec le milieu nutritif et les cellules.

Suivant une variante de l'invention, le support 1 peut comprendre des microbilles, par exemple de verre ou de polysaccharide insoluble ("Sephadex®") dispersées dans le milieu nutritif de culture. On bénéficie alors d'une meilleure transmission des éléments actifs, lors de l'application de la composition sur la peau. Comme le développement cellulaire, pendant la culture, a lieu surtout sur les microparticules, on peut séparer le liquide nutritif ce qui laisse pratiquement une pâte pour l'application sur la peau.

Dans le cas de cette variante de l'invention, le mode préféré de préparation de la composition cosmétique consiste à disperser d'abord les particules dans le liquide nutritif choisi, ensuite ensemencer celui-ci avec des cellules, notamment des fibroblastes, et laisser les cellules se multiplier. Le conditionnement de la culture, ainsi obtenue peut se faire alors - bien entendu à froid - selon un des modes décrits plus haut, l'ensemble ayant été conservé avec un excès de liquide. Il est toutefois recommandable de séparer le liquide des microbilles avant leur application sur la peu d'un sujet.

En plus des formes décrites, la nouvelle composition peut également être employée sous la forme de pâte ou de pommade. On disperse alors, dans la culture terminée et refroidie, en épaississant qui peut être par exemple un de ceux que l'on a cités plus haut, dans la description du support 1.

**Revendications**

1. Utilisation, comme composition cosmétique pour le traitement de la peau, d'un milieu nutritif de cellules de la peau, caractérise en ce qu'une culture de cellules de tissu conjonctif, particulièrement de fibroblastes, se trouve dans ce milieu nutritif.

2. Utilisation suivant la revendication 1, caractérisé en ce que la majeure partie de cellules présentes proviennent de leur culture dans ledit milieu.

3. Utilisation suivant la revendication 1 ou 2, dans laquelle la culture formant la composition cosmétique contient $10^2$ à $10^8$, et en général $10^4$ à $10^8$ cellules par ml.

4. Article cosmétique pour le traitement de la peau, comprenant un milieu nutritif de cellules de la peau, lequel milieu renferme une culture de cellules de tissus conjonctif, particulièrement de fibroblastes, associé à un support plat à pourtour encollé, ledit milieu étant recouvert d'un film insoluble dans l'eau.

5. Article suivant la revendication 4, dont le support est en une matière collagénique, un polysaccharide, polyvinylpyrrolidone, polyéthylène glycol, cellulose, acétate de cellulose ou copolymère de polyvinyl-pyrrolidone-acétate de vinyle.

6. Article suivant la revendication 4 ou 5, dont le support est sous une forme spongieuse.

7. Article suivant une des revendications 4 à 6, dont le milieu nutritif de la culture est le milieu d'EAGLE modifié, DMEM, complété avec du sérum de veau foetal et des acides aminés, caractérisé en ce qu'il contient de la glutamine et du pyruvate de Na.

8. Article suivant une des revendications 4 à 6, dont le support (1) est placé dans un récipient (5) de faible profondeur.

9. Article cosmétique suivant une des revendications 4 à 8, caractérisé en ce que des microparticules, en particulier des microbilles de verre ou de polysaccharide insoluble, sont dispersées dans ledit milieu nutritif.

10. Composition cosmétique pour le traitement de la peau, comprenant un milieu nutritif de cellules de la peau, renfermant une culture de cellules de tissu conjonctif, particulièrement de fibroblastes, caractérisé en ce qu'elle renferme, en outre, dispersées en son sein, des microparticules, en particulier des microbilles de verre ou de polysaccharide insoluble.

11. Composition cosmétique pour le traitement de la peau comprenant un milieu nutritif de cellules de la peau renfermant une culture de cellules de tissu conjonctif, particulièrement de fibroblastes, caractérisé en ce qu'elle renferme en outre un épaississant choisi dans le groupe constitué par une matière collagénique, un polysaccharide, polyvinyl-pyrrolidone, polyéthylène-glycol, cellulose, acétate de cellulose ou copolymère de polyvinyl-pyrrolidone/acétate de vinyle.

12. Procédé de préparation de la composition suivant la revendication 10 par culture de fibroblastes dans un milieu nutritif, caractérisé en ce que des microparticules, notamment en verre ou en polysaccharide insoluble, sont dispersées dans ce milieu, avant l'ensemencement avec des cellules prélevées dans la peau, et que le liquide est séparé des microparticules qui servent de support à la composition.

**Claims**

1. Use as a cosmetic composition for treatment of the skin of a nutrient medium comprising skin cells, characterised in that a culture of conjunctive tissue cells, particularly fibroblasts, is included in the nutrient medium.

2. Use according to claim 1, characterised in that the major part of the cells present is derived from their culture in the medium.

3. Use according to claim 1 or 2, in which the culture forming the cosmetic composition contains $10^2$ to $10^8$ and in general $10^4$ to $10^8$ cells per ml.

4. Cosmetic article for treatment of the skin, comprising a nutrient medium of skin cells, which medium includes a culture of conjunctive tissue cells, particularly fibroblasts, associated with a flat support having an adhesive periphery, the medium being covered with a film insoluble in water.

5. Article according to claim 4, wherein the support is a collagenic material, a polysaccharide, polyvinyl pyrrolidone, polyethylene glycol, cellulose, cellulose acetate or a polyvinyl-pyrrolidone-vinyl acetate copolymer.

6. Article according to claim 4 or 5, wherein the support has a spongy form.

7. Article according to any of claims 4 to 6, wherein the nutrient medium of the culture is a modified Eagle medium, DMEM, completed with foetal calf serum and amino acids, characterised in that it contains glutamine and Na pyruvate.

8. Article according to any of claims 4 to 6, the support (1) of which is located in a receptacle (5) of shallow depth.

9. Cosmetic article according to any of claims 4 to 8, characterised in that microparticles, in particular microspheres of glass or insoluble polysaccharide, are dispersed in the nutrient medium.

10. Cosmetic composition for treatment of the skin, comprising a nutrient medium of skin cells; containing a culture of conjunctive tissue cells, particularly fibroblasts, characterised in that it also has microparticles dispersed within it, in particular microspheres of glass or of insoluble polysaccharide.

11. Cosmetic composition for treatment of the skin comprising a nutrient medium of skin cells containing a culture of conjunctive tissue cells, particularly fibroblasts, characterised in that it also contains a thickener selected from the group comprising a collagenic material, a polysaccharide, polyvinylpyrrolidone, polyethylene-glycol, cellulose, cellulose acetate or a polyvinyl-pyrrolidone-vinyl acetate copolymer.

12. Process of preparation of a composition according to claim 10 by the culture of fibroblasts in a nutrient medium, characterised in that microparticles, particularly of glass or insoluble polysaccharide, are dispersed in the medium before seeding with cells taken from the skin and in that the liquid is separated from the microparticles which serve as a support for the composition.

## Patentansprüche

1. Verwendung eines Nährmediums für Hautzellen als kosmetische Zusammensetzung für die Behandlung der Haut, dadurch gekennzeichnet, daß eine Kultur von Bindegewebszellen, insbesondere von Fibroblasten, sich in dem Nährmedium befindet.

2. Verwendung nach Anspruch 1, dadurch gekennzeichnet, daß der Hauptteil der vorhandenen Zellen aus ihrer Kultur in diesem Medium herrührt.

3. Verwendung nach Anspruch 1 oder 2, worin die die kosmetische Zusammensetzung bildende Kultur $10^2$ bis $10^8$ und im allgemeinen $10^4$ bis $10^8$ Zellen pro ml enthält.

4. Kosmetikartikel zur Behandlung der Haut, welcher ein Nährmedium für Hautzellen umfaßt, welches Medium eine Kultur von Bindegewebszellen, insbesondere von Fibroblasten, assoziiert mit einer Trägerplatte mit klebendem Rand, einschließt, wobei das Medium mit einer in Wasser unlöslichen Folie bedeckt ist.

5. Artikel nach Anspruch 4, dessen Träger aus einem Kollagenmaterial, ein Polysaccharid, Polyvinylpyrrolidon, Polyethylenglykol, Zellulose, Zelluloseacetat oder ein Polyvinylpyrrolidon-Vinylacetat-Copolymer ist.

6. Artikel nach Anspruch 4 oder 5, dessen Träger eine Schwammform aufweist.

7. Artikel nach einem der Ansprüche 4 bis 6, dessen Nährmedium der Kultur ein modifiziertes EAGLE-Medium, DMEM, vervollständigt mit fötalem Kälberserum und Aminosäuren ist, dadurch gekennzeichnet, daß es Glutamin und Na-Pyruvat enthält.

8. Artikel nach einem der Ansprüche 4 bis 6, dessen Träger (1) in einem Rezipienten (5) von geringer Tiefe angeordnet ist.

9. Kosmetikartikel nach einem der Ansprüche 4 bis 8, dadurch gekennzeichnet, daß Mikroteilchen, insbesondere Mikrokugeln aus Glas oder aus unlöslichem Polysaccharid, in diesem Nährmedium dispergiert sind.

10. Kosmetische Zusammensetzung zur Behandlung der Haut, welche ein Nährmedium für Hautzellen umfaßt und eine Kultur von Bindegewebszellen, insbesondere von Fibroblasten, einschließt, dadurch gekennzeichnet, daß sie unter anderem Mikroteilchen, insbesondere Mikrokugeln aus Glas oder aus unlöslichem Polysaccharid, in sich dispergiert einschließt.

11. Kosmetische Zusammensetzung zur Behandlung der Haut, welche ein Nährmedium für Hautzellen umfaßt und eine Kultur von Bindegewebszellen, insbesondere von Fibroblasten, einschließt, dadurch gekennzeichnet, daß sie unter anderem ein Verdickungsmittel, ausgewählt aus der aus einem Kollagenmaterial, einem Polysaccharid, Polyvinylpyrrolidon, Polyethylenglykol, Zellulose, Zelluloseacetat oder Polyvinylpyrrolidon-Vinylacetat-Copolymer gebildeten Gruppe, einschließt.

12. Verfahren zur Herstellung der Zusammensetzung nach Anspruch 10 durch Kultur von Fibroblasten in einem Nährmedium, dadurch gekennzeichnet, daß vor dem Beimpfen mit den entnommenen Hautzellen Mikroteilchen, insbesondere aus Glas oder einem unlöslichen Polysaccharid in dem Milieu dispergiert werden und daß die Flüssigkeit von den Mikroteilchen, die als Träger der Zusammensetzung dienen, getrennt wird.

Fig.1

Fig.2

Fig.3